# FASCICULE DE BREVET EUROPEEN

(11) **EP 0 485 280 B1**
(45) Date de publication et mention de la délivrance du brevet: **15.05.1996**
(21) Numéro de dépôt: 91402968.1
(22) Date de dépôt: 06.11.1991
(51) Int. Cl.: A61B 17/34

(54) **Instrument chirurgical formant trocart**
Chirurgischer Trokar
Surgical trocar

(30) Priorité: 06.11.1990 FR 9013713
(43) Date de publication de la demande: 13.05.1992
(73) Titulaire: ETHICON INC., Somerville New Jersey 08876 (US)
(72) Inventeur: Bilweis, Joseph, F-78560 Noisy le Roi (FR)
(74) Mandataire: Martin, Jean-Jacques

(56) Documents cités:
- EP-A- 0 135 364
- EP-A- 0 350 291
- US-A- 3 539 034

## Description

La présente invention concerne le domaine des instruments chirurgicaux.

La présente invention a pour but principal de proposer un nouvel instrument chirurgical, de type trocart, propre à faire des ponctions notamment pour opérer des aspirations ou des injections de fluide, soit encore pour l'introduction d'un cathéter.

Ce but principal est atteint selon la présente invention grâce à un instrument chirurgical répondant aux caractéristiques mentionnées en revendication 1.

Selon une autre caractéristique avantageuse de la présente invention, les moyens de blocage à commande manuelle comprennent une gachette articulée sur la canule est conçue pour venir en prise avec au moins un crantage ménagé sur la surface extérieure de l'aiguille.

Selon une autre caractéristique avantageuse de la présente invention, l'aiguille est sollicitée vers la position de repos par un organe élastique.

Le document EP-A-0350291 concerne un instrument chirurgical conforme au préambule de la revendication 1 annexée et comprenant une canule de trocart qui loge une aiguille destinée à perforer la peau d'un patient pour permettre la pénétration de la canule de trocart, laquelle aiguille est disposée dans un manchon protecteur également logé dans la canule de trocart. En outre, le document EP-A-0350291 propose des moyens permettant une avance incrémentielle contrôlée du manchon protecteur par rapport à la canule de trocart.

D'autres caractéristiques, buts et avantages de la présente invention apparaîtront à la lecture de la description détaillée qui va suivre, et en regard des dessins annexés donnés, à titre d'exemples non limitatifs et sur lesquels :
- la figure 1 représente une vue schématique en coupe axiale longitudinale d'un instrument chirurgical conforme à la présente invention, en position de repos,
- la figure 2 représente une vue similaire en coupe axiale longitudinale du même instrument chirurgical, en position de travail,
- la figure 3 représente une vue similaire en coupe axiale longitudinale du même instrument chirurgical, dans une position intermédiaire entre les positions de repos et de travail précitées, et
- la figure 4 représente une vue schématique en coupe longitudinale axiale d'une canule conforme à une variante de réalisation de la présente invention.

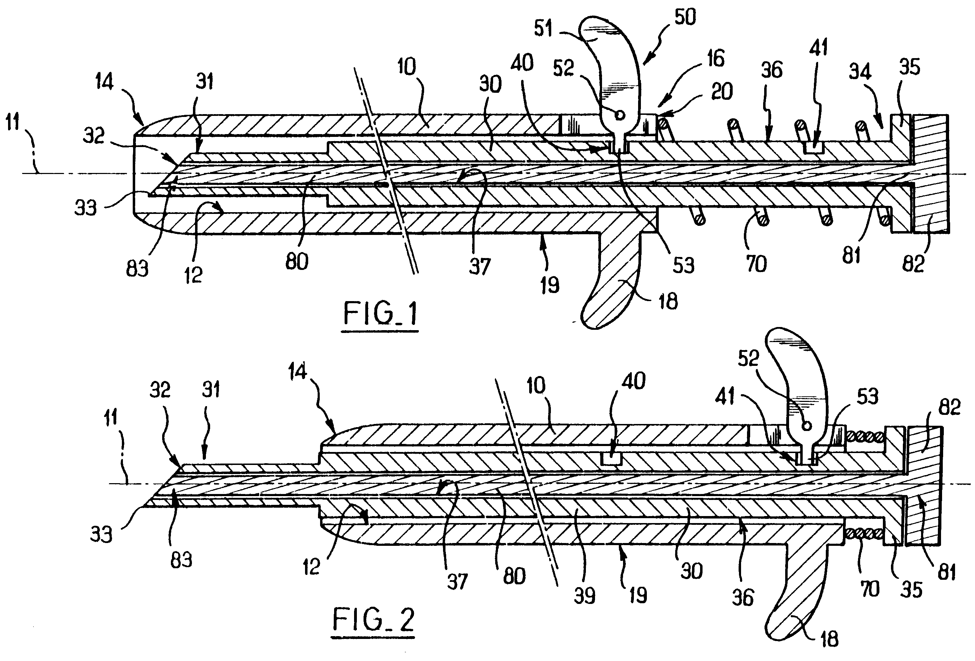

On aperçoit sur les figures 1 à 3 annexées un instrument chirurgical comprenant une canule 10, une aiguille creuse à extrémité tranchante 30, et des moyens de blocage à commande manuelle 50.

La lumière interne longitudinale de la canule 10 est référencée 12. Cette lumière est centrée sur l'axe longitudinal 11 de la canule.

L'extrémité proximale 14 de la canule est arrondie pour éviter de blesser les tissus.

La canule 10 est pourvue à son extrémité distale 16 de moyens de préhension 18. Ces moyens de préhension 18 peuvent faire l'objet de nombreux modes de réalisation. Il peut s'agir par exemple d'au moins un doigt 18 en saillie sur la surface externe 19 de la canule transversalement à l'axe 11, ou encore d'une collerette en saillie sur la surface extérieure 19 de la canule 10 centrée sur l'axe 11.

Les moyens de blocage 50 aptes à immobiliser à translation l'aiguille 30 par rapport à la canule 10 sont prévus au niveau de l'extrémité distale 16 de la canule 10.

L'aiguille 30 est engagée dans la lumière 12 de la canule 10. L'extrémité proximale 31 de l'aiguille 30 est effilée. De préférence, l'extrémité proximale 31 de l'aiguille 30 est délimitée par une face 32 oblique par rapport à l'axe 11. La face 32 définie ainsi un bord tranchant 33 à l'extrémité proximale 31 de l'aiguille.

L'aiguille 30 est munie à son extrémité distale 34 d'un disque 35 de plus grand évasement.

Selon le mode de réalisation particulier représenté sur les figures 1 à 3, et considéré actuellement comme préférentiel, les moyens de blocage à commande manuelle sont formés d'une gachette 51 aptes à venir en prise avec des crantages ménagés sur la surface extérieure 36 de l'aiguille.

Plus précisément, la gachette 51 est articulée sur la canule 10 autour d'un axe 52 qui s'étend transversalement à l'axe longitudinal 11 de la canule.

La gachette 51 peut être articulée autour de l'axe 52 par l'intermédiaire d'un tourillon engagé dans une chape formée dans le corps de la canule 10.

Le corps de la gachette 51 se prolonge au-delà de l'axe 52, en rapprochement de l'axe 11, par un doigt 53. Celui-ci est conçu pour être engagé dans l'un ou l'autre de deux crantages en creux référencés 40, 41 respectivement formés dans la surface extérieure 36 de l'aiguille 30.

Les deux crantages 40, 41 sont espacés axialement. Plus précisément, la position des deux crantages 40, 41 est telle que lorsque le doigt 53 de la gachette 51 est engagé dans le crantage 40 le plus proche de l'extrémité proximale 31 de l'aiguille 30, l'extrémité tranchante 33 de l'aiguille soit placée en retrait à l'intérieur de la canule 10. En revanche lorsque le doigt 53 est placé dans le second crantage 41 le plus proche de l'extrémité distale 34 de l'aiguille 30, comme représenté sur la figure 2, l'extrémité tranchante 33 de l'aiguille 30 fait largement saillie au-delà de l'extrémité proximale 14 de la canule 10.

De préférence, l'aiguille 30 est sollicitée vers sa position de repos représentée sur la figure 1 par un organe élastique.

De préférence cet organe élastique est formé d'un ressort hélicoïdal 70 engagé sur l'aiguille 30 entre le disque 35 prévu à l'extrémité distale de l'aiguille et la face 20 transversale à l'axe 11 délimitant l'extrémité distale 16 de la canule 10.

Comme représenté sur les figures annexées, l'aiguille 30 est creuse et possède un canal longitudinal traversant référencé 37. Une tige ou mandrin 80 peut être engagé temporairement dans le canal 37 de l'aiguille 30 pour obturer celui-ci. Pour autoriser le retrait de la tige 80, l'extrémité distale 81 de celle-ci est munie d'une mollette 82 ou tout moyen équivalent.

Par ailleurs, de préférence, l'extrémité proximale 83 de la tige est biseautée pour compléter sans discontinuité la face avant tranchante 32 de l'aiguille 30.

Comme indiqué précédemment lorsque, comme représenté sur la figure 1, le doigt 53 de la gachette 51 est placé dans le cran 40 de l'aiguille 30 le plus proche de l'extrémité proximale de celle-ci, l'aiguille 30 est immobilisée à translation par rapport à la canule dans une position de repos telle que l'extrémité tranchante 33 de l'aiguille 30 soit placée en retrait à l'intérieur de la canule 10. Dans cette position de repos représentée sur la figure 1, l'instrument chirurgical peut être approché de tissus, ou introduit dans une cavité, telle que la cavité abdominale, sans risque de blessure ou traumatisme pour les tissus ou organes environnants.

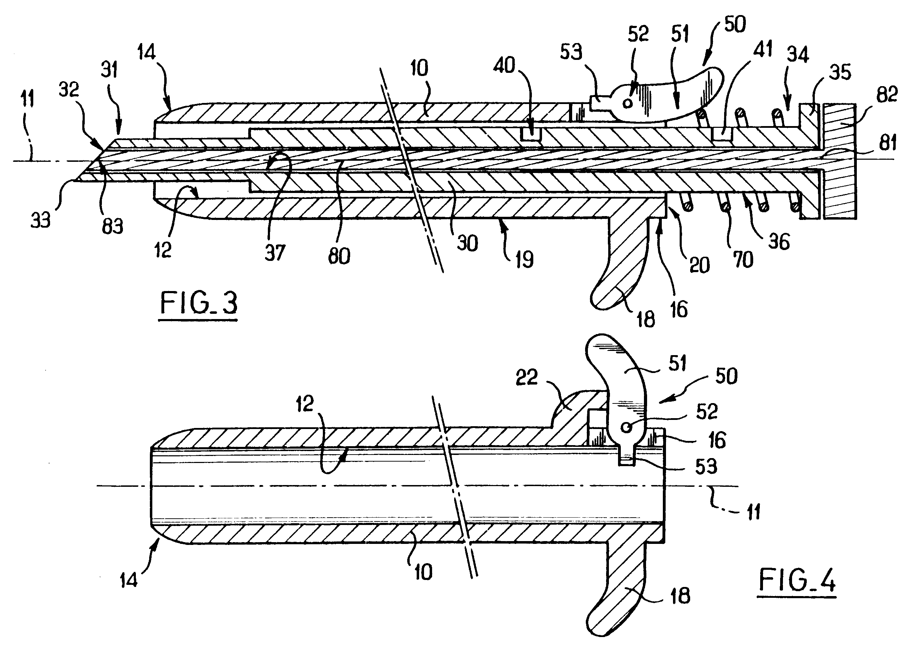

Pour autoriser l'utilisation de l'aiguille 30, il suffit de tirer la gachette 50 de sorte que le doigt 53 sorte du cran 40, comme représenté sur la figure 3. L'aiguille 30 peut être alors déplacée librement à translation par rapport à la canule 10. Si le chirurgien le souhaite l'aiguille 30 peut être immobilisée en saillie à l'extérieur de la canule 10 en engageant le doigt 53 dans le second cran 41. La position de travail ainsi obtenue est représentée sur la figure 2. Dans cette position l'extrémité tranchante 33 de l'aiguille fait largement saillie au-delà de l'extrémité proximale 14 de la canule 10.

Cette position de travail peut être utilisée pour réaliser des injections par l'intermédiaire du canal 37 de l'aiguille 30, ou encore des aspirations de fluide par l'intermédiaire du même canal 37, ou encore l'introduction d'un cathéter par l'intermédiaire de ce canal 37.

Lorsque la gachette 51 est relachée, le ressort 70 sollicite à nouveau l'aiguille 30 dans la position de repos représentée sur la figure 1. Une fois l'aiguille placée en retrait dans la canule 10, l'instrument peut être retiré sans risque de dommage pour les tissus environnants.

Dans l'état représenté sur la figure 3, intermédiaire entre la position de repos de la figure 1 et la position de travail de la figure 2, l'aiguille 30 peut être déplacée librement à translation en va et vient par rapport à la canule 10. Dans cet état, l'aiguille 30 peut être utilisée par exemple pour réaliser des ponctions.

Bien entendu lorsque le canal 37 de l'aiguille 30 est utilisé pour réaliser des injections, des aspirations, ou encore pour l'introduction d'un cathéter, la tige 80 doit être retirée.

En particulier, la structure des moyens de blocage à commande manuelle 50 peut faire l'objet de nombreuses variantes de réalisation.

Le cas échéant la canule 30 peut être pourvue de moyens interdisant la rotation de la gachette 51, dans le sens contraire des aiguilles d'une montre, à partir de sa position radiale en regard de l'axe 11 illustré sur les figures 1 et 2. Dans ce cas, l'aiguille 30 ne peut être rappelée de la position de travail vers la position de repos par le ressort 70 dès que la gachette 51 est relachée par l'utilisateur. Il faut alors en effet, solliciter l'aiguille 30 légèrement vers l'avant pour autoriser l'effacement du doigt 53 par rotation de la gachette 51 dans le sens des aiguilles d'une montre pour autoriser le rappel de l'aiguille en position de repos par le ressort 70. De tels moyens interdisant la rotation de la gachette 51 dans le sens contraire des aiguilles d'une montre à partir de la position radiale représentée sur les figures 1 et 2 peuvent être formées de butées au niveau de la chape portant la gachette 51 à articulation. On a représenté schématiquement de tels moyens sous forme d'une butée 22 sur la figure 4.

La représentation donnée sur les figures annexées n'est bien entendu que schématique. En particulier, les dimensions relatives de la canule 10 de l'aiguille 30 et des moyens de blocage 50, représentées schématiquement, ne sont aucunement limitatives.

Par ailleurs, on peut envisager de réaliser dans l'aiguille 30 un nombre de crantages 40, 41 supérieur à 2, afin de définir plus de deux positions stables de l'aiguille 30, par rapport à la canule 10. Cette disposition permet de contrôler avec précision l'amplitude en saillie de l'aiguille 30 par rapport à la canule 10.

Par ailleurs, la gachette 51 peut être associée à des moyens élastiques qui sollicitent celle-ci vers la position radiale par rapport à l'axe 11, telle que représentée sur les figures 1 et 2, dans laquelle le doigt de blocage 53 pénètre dans un cran 40 ou 41 de l'aiguille. Ces moyens élastiques peuvent être formés par exemple d'un ressort hélicoïdal engagé sur l'axe 52 et venant en prise, par ses extrémités, respectivement avec la canule 10 et la gachette 51.

## Revendications

1. Instrument chirurgical du type trocart comprenant une canule (10) et une aiguille (30) à extrémité tranchante engagée dans la canule (10) caractérisé par le fait que l'aiguille (30) est creuse, qu'une tige amovible (80) est engagée dans le canal (37) de l'aiguille (30) pour obturer sélectivement celui-ci et que l'instrument comprend en outre dès moyens de blocage (50) à commande manuelle aptes à immobiliser sélectivement l'aiguille (30) à translation, par rapport à la canule (10), lesquels moyens de blocage (50) à commande manuelle sont conçus pour définir deux positions de blocage entre l'aiguille (30) et la canule (10) : une position de repos dans laquelle l'extrémité tranchante (33) de l'aiguille (30) est placée dans la canule (10), et une seconde position, de travail, dans laquelle l'extrémité tranchante (33) de l'aiguille (30) est placée à l'extérieur de la canule (10).

2. Instrument selon la revendication 1, caractérisé par le fait que les moyens de blocage (50) à commande manuelle comprennent une gachette (51) articulée sur la canule (10) et conçue pour venir en prise avec deux crantages en creux (40, 41 ) ménagés dans la surface extérieure (36) de l'aiguille (30).

3. Instrument selon l'une des revendications 1 ou 2, caractérisé par le fait que l'aiguille (30) est sollicitée vers sa position de repos par un organe élastique (70).

4. Instrument selon la revendication 3, caractérisé par le fait que l'organe élastique (70) est un ressort hélicoïdal engagé entre l'extrémité distale (16) de la canule (10) et une butée (35) prévue à l'extrémité distale de l'aiguille (30).

5. Instrument selon l'une des revendications 1 à 4, caractérisé par le fait que la canule (10) est pourvue de moyens de préhension (18) à son extrémité distale.

## Claims

1. A surgical instrument of the trocar type, comprising a cannula (10), and a sharp-pointed needle (30) engaged in the cannula (10), the instrument being characterized by the fact that the needle (30) is hollow, that a removable rod (80) is engaged in the channel (37) of the needle (30) to close it selectively, and that the instrument further comprises manually controlled locking means (50) suitable for selectively locking the needle (30) in translation relative to the cannula (10), which manually controlled locking means (50) are designed to define two locking positions between the needle (30) and the cannula (10): a rest position in which the sharp point (33) of the needle (30) is located inside the cannula (10), and a working second position in which the sharp point (33) of the needle (30) is located outside the cannula (10).

2. An instrument according to claim 1, characterized by the fact that the manually controlled locking means (50) comprise a trigger (51) pivoted on the cannula (10) and designed to engage two recessed catches (40, 41) formed in the outside surface (36) of the needle (30).

3. An instrument according to claim 1 or 2, characterized by the fact that the needle (30) is urged towards its rest position by a resilient member (70).

4. An instrument according to claim 3, characterized by the fact that the resilient member (70) is a helical spring engaged between the distal end (16) of the cannula (10) and an abutment (35) provided at the distal end of the needle (30).

5. An instrument according to any one of claims 1 to 4, characterized by the fact that the cannula (10) is provided with handle means (18) at its distal end.

## Patentansprüche

1. Chirurgisches Instrument der Trokarart mit einer Kanüle (10) und einer Nadel (30) mit einem Schneidende, die in der Kanüle (10) eingesetzt ist, dadurch gekennzeichnet, daß die Nadel (30) hohl ist, daß eine herausnehmbare Stange (80) in dem Kanal (37) der Nadel (30) eingesetzt ist, um diesen gezielt zu verschließen, und daß das Instrument des weiteren manuell betätigte Sperrmittel (50) aufweist, die so ausgeführt sind, daß sie die Nadel (30) bezüglich der Kanüle (10) gezielt gegen eine Translationsbewegung sperren können, wobei die manuell betätigten Sperrmittel (50) so konfiguriert sind, daß sie zwei Sperrpositionen zwischen der Nadel (30) und der Kanüle (10) definieren: eine Ruheposition, in der das Schneidende (33) der Nadel (30) in der Kanüle (10) positioniert ist, und eine zweite Position, die Arbeitsposition, in der das Schneidende (33) der Nadel (30) außerhalb der Kanüle (10) positioniert ist.

2. Instrument nach Anspruch 1, dadurch gekennzeichnet, daß die manuell betätigten Sperrmittel (50) eine an der Kanüle (10) angelenkte Klinke (51) aufweisen, die so konfiguriert ist, daß sie mit zwei in der Außenfläche (36) der Nadel (30) ausgebildeten hohlen Einrastungen (40, 41) in Eingriff gebracht werden kann.

3. Instrument nach Anspruch 1 oder 2, dadurch gekennzeichnet, daß die Nadel (30) von einem elastischen Glied (70) zur Ruheposition hin vorgespannt ist.

4. Instrument nach Anspruch 3, dadurch gekennzeichnet, daß es sich bei dem elastischen Glied (70) um eine Schraubenfeder handelt, die zwischen dem distalen Ende (16) der Kanüle (10) und einem am distalen Ende der Nadel (30) vorgesehenen Anschlag (35) eingesetzt ist.

5. Instrument nach einem der Ansprüche 1 bis 4, dadurch gekennzeichnet, daß die Kanüle (10) an ihrem distalen Ende mit Greifmitteln (18) versehen ist.
